# EUROPEAN PATENT APPLICATION

(11) **EP 1 690 540 A1**
(43) Date of publication of application: **16.08.2006**
(21) Application number: 05290332.5
(22) Date of filing: 15.02.2005
(51) Int. Cl.: A61K 31/519, A61P 25/18, A61K 9/36

(54) **Composition comprising ocaperidone**

(71) Applicant: NEURO3D, 68058 Mulhouse Cedex 2 (FR)
(72) Inventor: Khanna, Satish, 4103 Bottmingen (CH); Mondadori, Cesare, 4153 Reinach (CH); Biondi, Stefano, 68400 Riedisheim (FR); Demailly, Arnold, 68200 Mulhouse (FR); Paparin, Jean-Laurent, 68720 Zillisheim (FR)
(74) Representative: Tezier Herman, Béatrice

(57) **Abstract**

The present invention relates to a composition comprising ocaperidone as an active substance and an effective amount of water-soluble polymers to increase solubility of ocaperidone. The present invention further relates to a therapeutic system for ocaperidone with a compartment for the drug formulation comprising said composition, and to a process for the preparation thereof as well as to the therapeutic use of said composition as antipsychotic drug. The present invention also relates to the solubilisation of ocaperidone in an aqueous medium with the aid of water soluble swellable polymers.

## Description

### TECHNICAL FIELD OF THE INVENTION

The present invention relates to a composition comprising ocaperidone as an active substance and an effective amount of water-soluble polymers to increase solubility of ocaperidone. The present invention further relates to a therapeutic system for ocaperidone with a compartment for the drug formulation comprising said composition, and to a process for the preparation thereof as well as to the therapeutic use of said composition as antipsychotic drug. The present invention also relates to the solubilisation of ocaperidone in an aqueous medium with the aid of water-soluble swellable polymers.

### BACKGROUND OF THE INVENTION

The most promising, common and convenient route of administering a drug delivery is considered to be the oral route. The optimal physico-chemical properties such as adequate lipophilicity and solubility in aqueous buffers of pH between 2 and 8 to allow high transcellular absorption following oral administration are well established. Furthermore, the characteristics of the dosage form are also of equal importance for a good and uniform bioavailability, because they can affect the dissolution and thereby the absorption properties of the drug. In general, the drug administered as a solution give faster and more complete absorption and thereby a better therapeutic performance. This is particularly the case for poorly water soluble drugs.

Ocaperidone (3-[2-[4-(6-fluoro-1,2-benzisoxazol-3-yl)-1-piperidinyl]ethyl]ethyl]-2,9-dimethyl-4H-pyrido[1,2-a]pyrimidin-4-one) is a well known antipsychotic drug (EP 196 132; EP 453 042). However, ocaperidone treatment generates side effects, more particularly the hyperprolactinaemia and the extrapyramidal side effects. These side-effects are due essentially to a large variability of ocaperidone plasma concentrations. Any means allowing to decrease or to abolish these side effects would be of great interest.
Ocaperidone is virtually insoluble in water. Its water solubility is less or equal to 0.007 mg per mL. The solubility in the buffers of gastro-intestinal fluids is also quite low; particularly in the buffers of pH 3.0 and above it lies in the same range as that of water. In order to be absorbed uniformly from the gastrointestinal tract after oral administration, the drug must be dissolved and must remain in the solution for absorption. The pH of the gastrointestinal tract varies from pH 1 to 5 in the stomach and pH 4 to 7.5 in the intestine. There is also known significant variation of the intestinal pH on an intra- and inter-individual basis in the general population. This variation is mostly attributed to factors such as gastro-intestinal motility, concomitantly administered food or drug and food habits etc.

Since in general the intestine is the optimal site for drug absorption, a change or variation in the pH at this site is likely to alter the rate and possibly extent of absorption of ocaperidone. This alteration in pH will thus affect the solubility of the drug in question and subsequently lead to variable bioavailability.
Bioavailability studies performed with conventional oral dosage forms of ocaperidone in animals as well as in humans revealed a significant high variability within a patient and also between patients. One of the objects of the invention is to overcome these drawbacks observed with conventional dosage forms.
Keeping this in mind, developing a drug delivery system whereby ocaperidone is released in a dissolved state was considered by the inventors to be of interest. As the drug releases in dissolved state, it will be available for absorption and thereby leading to less variability. An osmotic dosage form, from which the release is independent of surrounding pH, was also considered by the inventors to be the system of choice.

Theeuwes and Higuchi in US Patent Nos. 3,845,770 and 3,916,899 disclosed an advance in the delivery of therapeutic agents using an osmotic system comprising of a semi-permeable wall that surrounds a compartment containing an active agent. In this system the wall is permeable to the passage of an external fluid and impermeable to the passage of the therapeutic agent solution. An aqueous solution containing a therapeutic agent is delivered through a passageway in the wall. These systems are effective for delivering a therapeutic agent that is soluble in the fluid and exhibits an osmotic pressure gradient across the semi-permeable wall against the external fluid. The systems disclosed in these patents are however unsuitable for therapeutic agents having low solubility in water because the osmotic pressure generated by such an agent on its own is too low to cause release of the agent formulation from the core.

Theeuwes subsequently disclosed in US patent No. 4,111,202 an osmotic device with enhanced ability for delivering therapeutic agents insoluble in the aqueous fluid. The osmotic device of this patent has a therapeutic agent compartment and an osmotic diving agent compartment (or osmogent compartment), also called a push compartment. These two compartments are separated from each other. The fluid imbibed through the semi-permeable wall into the osmogent compartment causes the compartment to increase in volume. This pushes against the therapeutic agent compartment, thereby delivering the suspension of therapeutic agent through the passageway of the osmotic device to the external environment.

Khanna provides in US patent No 4,857,336 a single compartment osmotic dispensing device for delivering therapeutic agents with low solubility in aqueous and biological fluids. The osmotic device of this patent comprises a semi-permeable wall surrounding a compartment containing a therapeutic agent and expandable hydrogels. Upon uptake of external fluid, the hydrogel expands and thereby the suspension of the therapeutic agent formed within the cavity is dispensed through the passageway of the device. Subsequently this principle has been applied to many therapeutic agents with low solubility. However, in all cases the suspension was delivered from the system into the gastro-intestinal tract.

The inventors have surprisingly found that the solubility of ocaperidone in water can be enhanced by addition of specific water soluble swellable polymers suitable for the development of pharmaceutical compositions, in particular oral or rectal osmotic systems. The aqueous solution of ocaperidone delivered from such a system can overcome the above identified issues associated with this active substance such as inter- and intra- individual variability in the bioavailability.

In addition, this invention of improvement in solubility of ocaperidone in aqueous medium by specific polymers is not limited to its application for oral osmotic system. This property can also be used for other dosage forms, in particular oral dosage forms, such as a buccal form.

### SUMMARY OF THE INVENTION

It is an object of the present invention to provide a therapeutic system for ocaperidone from which despite its very low solubility in water (0.09 mg/mL) it is dispensed/released in the surrounding intestinal environment in a dissolved state. This invention eliminates the effect of surrounding pH on the solubility of ocaperidone.
In addition, this improvement in solubility makes this therapeutic system to function appropriately from the bioavailability point of view and overcomes the inter- and intra-individual variability.

In a first aspect, the invention concerns a composition comprising ocaperidone, as an active ingredient, and an effective amount of water-soluble swellable polymers.

In a particular aspect of the invention, said composition is a pharmaceutical composition further comprising a pharmaceutically acceptable carrier.

In another particular aspect, the invention relates to a solid pharmaceutical dosage for administration, in particular oral or rectal administration, in the form of a coated and/or laminated system for the administration of ocaperidone. The solid pharmaceutical dosage of the invention comprises :
(a) a wall made of a material which is permeable to water and impermeable to the compartments of the ocaperidone-containing core,
(b) a core containing the composition as defined above, i.e., ocaperidone as drug in the presence of water swellable hydrophilic polymer(s), and a water-soluble compound for inducing osmosis and optionally further pharmaceutically acceptable excipients, and
(c) a passageway through the wall (a) for delivering the core components to the environmental body fluid.

The invention further relates to a process for the preparation of said therapeutic system.

Moreover, the invention deals with the use of said composition to prepare a pharmaceutical composition to treat psychosis and methods for treating psychosis by administering such composition to a subject in need of such treatment.

### DETAILED DESCRIPTION OF THE INVENTION

The composition comprising ocaperidone, as an active ingredient, and an effective amount of water-soluble swellable polymers can be a pharmaceutical composition, in particular an oral or rectal pharmaceutical composition. This composition can be used in a specific system which is a solid pharmaceutical dosage form. In this particular aspect, the system is sized, shaped and adapted as a dosage form for delivering drug to the rectal tract or more preferably to the gastroinstestinal tract.

Therefore, the invention describes a pharmaceutical composition, in particular a solid pharmaceutical dosage form, from which ocaperidone, as active ingredients, is released at a controlled rate and conditions independently of the pH, i.e. of the concentration of hydrogen ions and hydroxyl ions, and/or other ions and also of enzymes of the body fluid.

The definitions and terms employed throughout this specification have the following preferred meanings within the scope of the description of this invention.
The wall (a) made of material which is permeable to water and impermeable to the components of the active substance core may be understood as being a semi-permeable membrane which is permeable to the passage of water but substantially impermeable to the passage of components present in the core of the dosage form, e.g. ocaperidone, swellable polymer, osmotic agent and the like.

The pharmaceutical composition, in particular the therapeutic system, according to the present invention, comprises a very low dose of ocaperidone. Preferably, the amount of ocaperidone is from 0.05 to 5 % by weight of the total weight of the composition (e.g. system), more preferably less than 2 %. In a particular preferred embodiment, the amount of ocaperidone is about or is less than 1 % of the total weight of the composition (e.g. system). In another particular preferred embodiment, the amount of ocaperidone is less than 1 % of the total weight of the composition (e.g. system), more preferably about 0.1 %. Indeed, the pharmaceutical composition (e.g., therapeutic system) comprises advantageously less than 5 mg of ocaperidone, preferably less than 3 mg, whereas the total weight of the pharmaceutical composition (e.g. therapeutic system) is from 150 to 250 mg. In a particular preferred embodiment, the pharmaceutical composition (e.g. therapeutic system) comprises between about 0.1 to about 3 mg of ocaperidone.

Ocaperidone is preferably used in a finely particulate form for the pharmaceutical composition (e.g. therapeutic system) of the present invention. Indeed, as the dose of ocaperidone is very low, the finely particulate form allows uniformity within the batch to be achieved. The expression "finely particulate form" will be understood as comprising micronised anhydrous and/or micronised crystalline forms. The particle size must be chosen such that it permits enhanced dissolution of the sparingly soluble ocaperidone in the water permeated in the pharmaceutical composition (e.g. therapeutic system) and thereby the release through the orifice of the wall is ensured. In a preferred embodiment of the dosage form of this invention, crystals of ocaperidone having an average particle size smaller than 100 µm, preferably smaller than 20 µm, and more preferably smaller than 10 µm are used (measured with a microscope).

The swellable hydrophilic (or water-soluble) polymer present in the composition of the invention is an excipient that interacts with water or the aqueous fluid, swells, and expands to a state of equilibrium. The swellable hydrophilic polymers have the ability to absorb large amounts of water. In addition, these polymers have also the quality to induce the pressure necessary for the therapeutic system to function. As the semi-permeable wall (a) is rigid, or at least of only limited elasticity, the pressure induced by expansion is compensated for by release of the material present in the core through the passageway (c) provided in the semi-permeable wall.

Examples of suitable swellable hydrophilic polymers are polymers which may be uncrosslinked or in which, if crosslinked, the crosslinks are formed by covalent or ionic bonds. The polymer retains the ability to swell in the presence of fluids without dissolving completely in the fluid when crosslinked. The polymers can be of plant, animal, mineral or synthetic origin.

Polymers which are particularly suitable for use in the practice of this invention are the known hydrogels of the swellable cellulose ether type, e.g. methyl cellulose, hydroxyethyl cellulose or hydroxypropyl methylcellulose, hydroxypropyl cellulose preferably having a molecular weight higher than 10,000, or mixtures of said swellable hydrophilic polymers. Otherwise, the present invention also considers the water-soluble polyvinylamides and polyvinylpyrrolidone as suitable swellable polymers.
In a preferred embodiment of the invention, the swellable hydrophilic polymer is selected from the group consisting of hydroxyethyl cellulose, hydroxypropyl cellulose, hydroxypropyl methylcellulose, polyvinylpyrrolidone, and a combination thereof. In a particularly preferred embodiment of the invention, a mixture of the copolymer of hydroxyethyl cellulose and hydroxypropyl methylcellulose is used as swellable hydrophilic polymers. Preferably, said polymers have a molecular weight higher than 10,000.

The composition, and more particularly core (b) of the therapeutic system, can contain 5-50, more preferably 5-30%, by weight of swellable hydrophilic polymers, and most preferably about 15% (i.e., ± 2%), based on the total weight of the composition (and more particularly of the therapeutic system).

The composition, and more particularly core (b) of the therapeutic system, present a weight ratio ocaperidone:swellable hydrophilic polymers ranging from 1:10 to 1:1000, preferably from 1:50 to 1:500, and most preferably 1:100 to 1:300. This ratios apply more particularly for polyvinylpyrrolidone.

In a particular embodiment, the composition of the invention may further comprise other swellable polymers, and especially swellable cross-linked polymers, and not necessarly water-soluble polymers, such as ion-exchange resins.

In a preferred embodiment of the invention, swellable cross-linked polymers such as polyvinylpolypyrrolidone, sodium salt of carboxymethylcellulose, and ion-exchange resins may be used. In presence of water, these adjuncts conveniently swell without dissolving many folds of their volume. A preferred example is a crospovidon e.g. polyplasdone XL and Kollidone CL (Fielder loc cit p. 1245). The composition according to the invention (and more particularly core (b) of the therapeutic system) can contain 20-80% by weight of swellable cross-linked polymers, preferably about 50%, based on the total weight of the pharmaceutical composition (and more particularly the therapeutic system).

In addition to that finely divided particulate form, ocaperidone can be complexed with a weak or strong cationic resin, in particular weak cationic resin, such as Amberlite™ IRP 64, Amberlite™ IRP 89 (methacrylic acid- Divinylbenzene). Since the amount of ocaperidone in the composition is quite low, a dilution by loading onto weak cation resin can be used to achieve the appropriate content uniformity from system to system conveniently. The weight ratio ocaperidone:resin is preferably from 0.5:50 to 2:30, more preferably from 0.7:100 to 1.3:30.

In a particular embodiment, the composition of the invention may further comprise other polymers. Such polymers which can also be particularly suitable for use in the practice of this invention are the known cyclodextrins. The term "cyclodextrin" refers to any known natural cyclodextrin as well as substituted and unsubstituted analogs and any derivatives thereof. Examples of suitable derivatives include methyl-beta-cyclodextrin, hydroxyethyl-betacyclodextrin and hydroxypropyl-beta-cyclodextrin.

Generally, these substances are cyclic oligosaccharides with the ability to form inclusion complexes with a variety of materials. Their ring size is typically from 6 to 12 glucose units, preferably 6, 7 or 8 glucose units. Preferred rings include alpha-cyclodextrin, beta-cyclodextrin and gamma-cyclodextrin, respectively.

In a particular aspect, the composition comprises the active drug alone or drug physically or chemically bound to an appropriate adjunct like ion-exchange resin, at least one swellable water-soluble polymer at least one osmosis-inducing compound, and eventually one or more pharmaceutically acceptable excipients. This composition is particularly suitable to be used as the core (b) as defined above. The core can be coated with a semi-permeable membrane and a passageway (orifice) of an appropriate size is drilled therein.

After passage through the water of the ambient body fluid, the drug in a dissolved form is released in a uniform and controlled way through the orifice in the whole anal or more preferably in the whole gastro-intestinal tract. This system leads to the least fluctuation in the release rate in the anal or gastro-intestinal tract.

Suitable materials for forming the semi-permeable wall are e.g. the polymeric semi-permeable materials described in the literature, e.g. in U.S. Pat. Nos. 3,916,899 and 3,977,404, and which are not metabolised in the gastrointestinal tract, i.e. which are excreted intact. For example, it is possible to use acetylated cellulose derivatives (cellulose esters) which are substituted by one to three acetyl groups or by one or two acetyl groups and a further acyl radical other than acetyl, e.g. cellulose acetate, cellulose triacetate, agar acetate, amylose acetate, cellulose acetate ethyl carbamate, cellulose acetate phthalate, cellulose acetate methyl carbamate, cellulose acetate succinate, cellulose acetate dimethylaminoacetate, cellulose acetate ethyl carbonate, cellulose acetate chloroacetate, cellulose acetate ethyl oxalate, cellulose acetate methyl sulfonate, cellulose acetate butyl sulfonate, cellulose acetate propionate, cellulose acetate diethylaminoacetate, cellulose acetate octate, cellulose acetate laurate, cellulose acetate p-toluenesulfonate, cellulose acetate butyrate and other cellulose acetate derivatives. Suitable semi-permeable membrane materials are also polymeric epoxides, copolymers of alkylene oxides and alkyl glycidyl ethers, polyglycols or polylactic acid derivatives and further derivatives thereof. It is also possible to use mixtures, e.g. of water-insoluble acrylates (e.g. the copolymer of ethyl acrylate and methyl methacrylate). In a preferred embodiment, the semi-permeable wall comprises cellulose acetate, hydroxypropyl methylcellulose and polyethylene glycol. Preferably, cellulose acetate is a mixture of cellulose acetate containing 32.0 % and 39.8 % acetyl groups (% expressed by weight).

In a particular embodiment, the semi-permeable wall (or coating) can be coated partially or totally by a composition comprising ocaperidone. This coating of ocaperidone allows an immediate release of the drug to be achieved. In a preferred embodiment, the composition (corresponding to a coating of the semi-permeable wall) comprises ocaperidone, as an active ingredient, and an effective amount of water-soluble swellable polymers, as defined above.

The core in addition to the swellable hydrophilic polymers comprises water-soluble compounds for inducing osmosis. Water-soluble compounds suitable for inducing osmosis are, in principle, all pharmacologically acceptable water-soluble compounds, e.g. the water-soluble excipients referred to in pharmacopeias or in "Hager" as well as in Remington's Pharmaceutical Sciences. Especially suitable are pharmaceutically acceptable water-soluble salts of inorganic or organic acids or nonionic organic compounds of particularly high water solubility, e.g. carbohydrates such as sugar, sugar alcohols, or amino acids. A combination of these osmosis inducing agents may also be used.

Examples of such water-soluble compounds for inducing osmosis are: inorganic salts such as magnesium chloride or magnesium sulfate, lithium, sodium or potassium chloride, lithium, sodium or potassium hydrogen or dihydrogen phosphate, salts of organic acids such as sodium or potassium acetate, magnesium succinate, sodium benzoate, sodium citrate or sodium ascorbate; organic acids such as citric acid, tartartic acid, succinic acid etc; carbohydrates such as sorbitol or mannitol (hexite), arabinose, ribose or xylose (pentosene), glucose, fructose, galactose or mannose (hexosene), sucrose, maltose or lactose (disaccharides) or raffinose (trisaccharides); water-soluble amino acids such as glycine, leucine, alanine or methionine, urea and the like, and mixtures thereof. In a preferred embodiment, these water-soluble compounds are present in the core in amounts by weight of 20 to 35 %. In a more preferred embodiment, the water-soluble compound is present in the core in amounts by weight of about 30 % based on the total weight of the therapeutic system. Preferred water-soluble compounds are selected in the group consisting of sodium chloride, mannitol, dextroses and dextrate. In a most preferred embodiment, the water-soluble compound is sodium chloride.

In addition to containing the water-soluble compounds for inducing osmosis and the swellable hydrophilic polymers, the pharmaceutical composition, and more particularly core (b), may contain further pharmaceutically acceptable excipients.

Preferred additional excipients are surface-active compounds also known as surfactants, e.g. anionic surfactants of the alkylsulfate type such as sodium, potassium or magnesium n-dodecylsulfate, n-tetradecylsulfate, n-hexadecylsulfate or n-octadecylsulfate; of the alkyl ether sulfate type, e.g. sodium, potassium or magnesium n-dodecyloxyethyl sulfate, n-tetradecyloxyethyl sulfate, n-hexadecyloxyethyl sulfate or n-octadecyloxyethyl sulfate; or of the alkylsulfonate, type e.g. sodium, potassium or magnesium n-dodecanesulfonate, e.g. sodium, potassium or magnesium n-tetradecanesulfonate, n-hexadecanesulfonate or n-octadecanesulfonate. In a preferred embodiment, the core comprises sodium lauryl sulfate. Further suitable surfactants are nonionic surfactants of the fatty acid polyhydroxy alcohol ester type such as sorbitan monolaurate, sorbitan monooleate, sorbitan monostearate or sorbitan monopalmitate, sorbitan tristearate or triolate, polyethylene glycol fatty acid ester such as polyoxyethyl stearate, polyethylene glycol 400 stearate, polyethylene glycol 2000 stearate, preferably ethylene oxide/propylene oxide block polymers of the Pluronics™ (BWC) or Synperonic™ (ICI) type.
Preferably, the weight ratio of surfactants to the composition (more particularly the core) weight of the invention is 1:100 to about 1:500.
Further excipients are those customarily used in tableting for the preparation of granulates, e.g. binders, lubricants, glidants, dispersants, fillers and the like. Thus it is possible to use conventional auxiliaries such as i) natural , , starches, e.g. potato starch, corn starch ii) modified starches used for direct compression, for example starx ® 1500, carboxymethylstarches, sodium starch glycolate available as Primojel®, explosol® and (iii) starch derivatives such as amylose or amylopectin, or iv) celluloses such as cross-linked sodium carboxy methylcellulose available as Ac-disol®, Primellose®, and especially microcrystalline cellulose.
Microcrystalline cellulose is preferably present to make the core of desired size. It is used as a filler. Examples include the Avicel of different types (FMC corporation) for example of the types Avicel PH101, 102, 105, RC581 or RC 591 (fielder p 216) Emocel type (Mendell Corp.), Elceme Type (Degussa), Filtrak® type and Heweten® type. Hence, the present invention provides more preferably an oral dosage form comprising microcrystalline cellulose (as a filler).

Oral dosage form may also contain colloidal silicas e.g. Aerosil ® 200 (Fielder p 17) (as glidant). Examples of other glidants include silica, magnesium trisilicate, powdered cellulose, starch and talc.
Magnesium stearate is a preferred excipient. It may function as a lubricant. Examples of other lubricants include calcium stearate, zinc stearate, talc, polyethylene glycol, stearic acid and sodium benzoate. Combination of lubricants may also be used.

The expression "passageway through the walls (c) for delivering the components present in the core to the environmental aqueous body fluid" encompasses means and methods suitable for releasing the drug formulation from the core of the therapeutic system. The expression comprises passages, orifices, bores, apertures and the like through the wall (a) acting as semi-permeable membrane which establish a connection between the surface of the wall and the core. In one embodiment of the invention, two or more passageways can be provided, which may be located anywhere in the system. The passageway can also be made by mechanical rupture of the layers while the system is in use. The passageway has a minimum diameter which is dependent on the viscosity of the gel formed inside the system in the permeated water. In particular, the diameter of the passageway must be such that the most viscous gel can also be pumped out unhindered. The maximum diameter is also approximately fixed. It may only be so large that the entry of the aqueous body fluid into the therapeutic system by convection is avoided. An exact description of the passageway and of the maximum and minimum dimensions will be found in U.S. Pat. Nos. 3,485,770 and 3,916,899 and in the drawings pertaining thereto.

The therapeutic system may differ in shape and be e.g. round, oval, tubular and the like, and may also differ in size, depending on the amount of fill material. The system is sized, shaped and adapted as a dosage form for delivering drug to the rectal tract or more preferably to the gastroinstestinal tract. Furthermore, the therapeutic system can be transparent, colourless or coloured, so as to impart an individual appearance or immediate identification to the product.

The present invention relates preferably to a, preferably oral or rectal, therapeutic system comprising:
(a) a wall comprising acetylated cellulose, e.g. cellulose acetate, which is permeable to water but impermeable to the components of the drug-containing core and to the ions present in body fluids, e.g. gastric or intestinal juices,
(b) a core containing ocaperidone as drug, a mixture of hydroxypropylmethyl cellulose and hydroxyethylcellulose as swellable hydrophilic polymers, sodium or potassium chloride, glucose or mannitol as agent for inducing osmosis, as well as further pharmaceutically acceptable excipients, such as surfactants or lubricants, and
(c) a passageway through the wall (a) for delivering the components present in the core to the environmental aqueous body fluid.

According to a particular embodiment, the therapeutic system as defined above further comprises a partial or total coating on the wall comprising ocaperidone and water soluble swellable polymers as defined above, in particular hydroxypropylmethyl cellulose, hydroxyethylcellulose, polyvinyl pyrrolidone or a mixture thereof.

Ocaperidone is a potent antagonist of neurotransmitters and in particular of dopamine. Antagonizing said neurotransmitter suppresses a variety of phenomena induced by the release, in particular the excessive release, of dopamine. Central dopamine receptor antagonists are known to have neuroleptic properties, for example, they counteract the positive symptoms of schizophrenia, e.g. hallucinations, delusional thinking, severe excitement and unusual behaviour. Therapeutic indications for using the present composition therefore are mainly in the CNS (Central Nervous System) area, particularly as potent antipsychotic formulation and especially as formulation useful in treating acute psychoses. The present system is particularly effective in treating psychiatric patients suffering from severe agitation and in need of rapid restabilization. The present system is also efficacious in treating patients not responding or responding poorly to administration of other neuroleptics such as haloperidol or risperidone. Ocaperidone is also known to show central serotonin antagonism. Central acting serotonin antagonists appear to improve the negative symptoms of schizophrenia, e.g. anergy, apathy, social withdrawal and depressive mood, and also appear to reduce the incidence of extrapyramidal side-effects during maintenance therapy with classical neuroleptics, i.e. dopamine antagonists. Combined dopamine-serotonin antagonists are especially interesting as they offer relief of both the positive and negative symptoms of schizophrenia. The therapeutic system of the invention has therefore valuable pharmacological properties and can be used in particular for the treatment of psychotic conditions, in particular schizophrenia (positive and negative symptoms thereof), mania, obsessive compulsive disorders, tourette syndrome, anxiety and bipolar depression.

The use of the above-described composition or system for preparing a pharmaceutical composition or system for the treatment of the above identified disorders constitutes a further object of the invention.

The present invention provides also a method of treating warm-blooded animals, in particular humans, suffering from such diseases, in particular psychotic diseases, said method comprising the systemic administration of an effective amount of the composition or system according to the invention, effective in treating diseases associated with the release of neurotransmitters, in particular psychotic diseases. Those of skill in the treatment of such diseases could easily determine the effective amount to treat such diseases. In general, it is contemplated that an effective antipsychotic amount of the active ingredient would be from about 0.0025 to about 4 mg/kg of body weight, preferably from about 0.01 mg/kg to about 1 mg/kg body weight, more preferably from about 0.02 mg/kg to about 0.10 mg/kg body weight. The required dose may advantageously be administered as one, two, three or more times at appropriate intervals throughout the day.

The therapeutic system of the invention is prepared by methods which are known per se, e.g. by triturating the components of the core together and compressing them, coating the core with a semi-permeable wall and providing an appropriate size of a passageway through said semi-permeable wall also known as an orifice. Coating of the semi-permeable wall can also be implemented with a composition comprising ocaperidone as described above. Furthermore, if desired for immediate availability of drug after intake of dosage form, a part of ocaperidone may be coated on the top of semi-permeable membrane.

The following Examples illustrate the invention in more detail without limiting the scope thereof. The percentages are expressed by weight unless specified otherwise.

### EXAMPLES

Following is a description by way of examples only of compositions and processes of the invention. In all examples the compound of the invention is in extra fine grade.

### EXAMPLE 1:

### a.) Preparation cores containing 2.2 mg ocaperidone / system

**Composition for 1000 cores:**

| Ingredients | Amount in g |
|---|---|
| Ocaperidone | 2.20 |
| Microcrystalline cellulose | 100.00 |
| Hydroxypropylmethyl cellulose | 14.80 |
| Hydroxyethylcellulose 250L | 5.00 |
| Hydroxyethylcellulose 250H | 10.00 |
| Dextrate fine | 27.50 |
| Mannitol fine | 27.50 |
| Sodium lauryl sulphate | 1.00 |
| Magnesium stearate | 2.00 |
| **Total weight of mass** | 192.00 |
| Weight per weight | 192.00 mg |
| Diameter of core | 7.00 mm |

### Preparation of cores:

Ocaperidone was mixed with all excipients of core, except magnesium stearate using a geometrical mixing procedure. The mixture was subsequently passed through a sieve and blended using a bin-blender (Turbula) and the mix was passed once again through the sieve of 0.3 mm.
The premix was granulated with deionised water in a laboratory mixture. The resulted wet-granulated mass was screened using an appropriate size sieve and dried in a fluidized-bed dryer (Aeromatic Strea-1). The loss on drying for this mass was around 2.0%.
Dried granules were passed through a sieve of 0.8 mm mesh.
Magnesium stearate was screened through a sieve of 0.3 mm mesh and added to the above granules.
The final blend was mixed using a bin-blender e.g. Turbula.
The cores of film-coated shape were compressed using punch and die of 7.00 mm diameter on a single punch machine.

### Coating of system

**Composition and preparation of lacquer**

| Ingredients | In % age |
|---|---|
| Cellulose acetate 320 | 73 |
| Cellulose acetate 398-10 | 17 |
| Hydroxypropyl methylcellulose 3cps | 5 |
| Polyethylene glycol 8000 | 5 |

The ratio of solid to solvent mixture in the lacquer is 5 to 95. The solvent-mixture consists of 90% to 10% of methylene chloride and methanol.
The solid contents of the above table were added to the solvent-mixture and stirred for sufficient time until the solid contents were completely dissolved.

### Coating and drilling process

500 cores prepared above were placed in a fluidised bed coater (Aeromatic strea-1). The lacquer was sprayed using an appropriate size of nozzle till around 40 mg solid material was deposited on each core.
The coated systems were dried in a drying chamber at around 40°C for 48 hours.

The dried systems were drilled mechanically with an orifice of 0.5 mm diameter and tested for their functionality and release rate properties.

### Functionality and release testing

In five individual beakers, approximately 200mL water was placed. These were then placed in a water-bath having 37°C. In each beaker, a system prepared above were placed and tested for the functionality.
All the systems pumped the active substance and excipients from the orifice and remained intact for 24 hours.

The release rate tested using USP2 method gave the following results.

| Time in hours | % age drug released |
|---|---|
| 2 | 6.2 |
| 4 | 26.5 |
| 6 | 51.5 |
| 8 | 66.6 |
| 10 | 75.9 |
| 12 | 81.6 |
| 18 | 92.8 |
| 24 | 106.9 |

### EXAMPLE 2:

### a.) Preparation containing 0.22 mg ocaperidone / system

**Composition for 1000 cores:**

| Ingredients | Amount in g |
|---|---|
| Ocaperidone | 0.22 |
| Microcrystalline cellulose | 59.78 |
| Polyvinyl pyrrolidone | 12.50 |
| Cross-linked carboxymethylcellulose Sodium | 60.00 |
| Sodium chloride fine | 50.00 |
| Sodium lauryl sulphate | 1.50 |
| Magnesium stearate | 2.00 |

| **Total weight of mass** | 186.00 |
|---|---|
| Weight per weight | 186.00 mg |
| Diameter of core | 7.00 mm |

### Preparation of cores:

The cores were prepared as mentioned in the example 1.

### Coating of system

**Composition and preparation of lacquer**

| Ingredients | In g |
|---|---|
| Cellulose acetate 320 | 6.80 |
| Cellulose acetate 398-10 | 29.20 |
| Hydroxypropyl methylcellulose 3cps | 2.00 |
| Polyethylene glycol 8000 | 2.00 |

The lacquer was prepared by dissolving the solid contents in a mixture of methylene chloride and methanol.

### Coating and drilling process

500 cores prepared above were placed in a fluidised bed coater (Aeromatic strea-1). The lacquer was sprayed using an appropriate size of nozzle till around 20 mg solid material was deposited on each core.
The coated systems were dried in a drying chamber at around 40°C for 48 hours.

The dried systems were drilled mechanically with an orifice of 0.5 mm diameter and tested for their functionality and release rate properties.

### Functionality testing

In five individual beakers, approximately 200mL water was placed. These were then placed in a water-bath having 37°C. In each beaker, a system prepared above were placed and tested for the functionality.

All the systems pumped the active substance and excipients from the orifice and remained intact for 24 hours.

### EXAMPLE 3:

### Preparation containing resinate equivalent to 0.22 mg ocaperidone / system

**Composition for 1000 cores:**

| Ingredients | Amount in g |
|---|---|
| Ocaperidone resinate | 8.00 |
| Microcrystalline cellulose | 64.00 |
| Polyvinyl pyrrolidone | 12.50 |
| Hydroxyethylcellulose 250L | 3.00 |
| Hydroxyethylcellulose 250H | 6.50 |
| Sodium chloride fine | 69.50 |
| Sodium lauryl sulphate | 0.50 |
| Magnesium stearate | 2.00 |
| **Total weight of mass** | 166.00 |
| Weight per weight | 166.0 mg |
| Diameter of core | 7.00 mm |

### a.) Preparation of resinate using Amberlite IRP 64

Dissolve 2.0 g of ocaperidone in 1 L ethanol. Add 1L of deionised water and stir. Add 70 g of acidic form of Amberlite IRP64 to the above solution. Heat the suspension to 40°C and stir for 24 hours or longer till the total drug from the solution almost disappears.
Filter the solid particle and wash these with water and subsequently with ethanol. Dry the resinate so obtained in a drying chamber till a constant weight is achieved.

### b.) Preparation of cores and coating:

Same methods and equipment were applied as described in example 1. The composition of the core is given above.
The composition of solid contents in lacquer used for coating consisted of 6.8 g cellulose acetate 320, 29.2 g of cellulose acetate 398, 2.0 g hydroxypropylmethyl cellulose and 2 g propylene glycol 8000. These substances were dissolved in methylene chloride and methanol mixture.
The coating and drying steps were same as described in above examples.
The dried systems were drilled mechanically with an orifice of 0.5 mm diameter and tested for their functionality and release rate properties.

### Release rate testing

The release rate was tested as described in the example 1 and average results (n=6) were as follows:

| Time in hours | % age drug released |
|---|---|
| 2 | 8.5 |
| 4 | 33.0 |
| 6 | 51.5 |
| 8 | 68.0 |
| 10 | 76.9 |
| 12 | 84.6 |
| 18 | 95.8 |
| 24 | 101.5 |

### EXAMPLE 4:

### Preparation containing 0.22 mg ocaperidone / system

| Ingredients | Amount (mg/system) |
|---|---|
| Core | |
| Ocaperidone | 0.22 |
| Hydroxypropylmethylcellulose | 12.00 |
| Hydroxyethylcellulose 250L | 5.00 |
| Hydroxyethylcellulose 250H | 10.00 |
| Mannitol fine | 77.25 |
| Dextrates hydrated | 77.25 |
| Sodium lauryl sulphate | 1.30 |
| Magnesium stearate | 2.00 |
| Total weight of core | 185.00 |
| Diameter of core | 7.00 mm |
| Coating (rate controlling) | |
| Cellulose acetate 320 | 17.0 % |
| Cellulose acetate 398 | 73.0% |
| Hydroxypropylmethylcellulose E15 | 5.0% |
| Polyethylene glycol 8000 | 5.0% |
| Total coating weight/system | 13.0 mg |
| Total weight of system | Approx. 198 mg |

### Functionality testing

The test was performed as mentioned under example 2 using 5 individual systems. All the systems pumped the active substance and excipients from the orifice and remained intact for 24 hours.

### EXAMPLE 5:

### Preparation containing 0.22 mg ocaperidone / system (with an ocaperidone coat)

| Ingredients | Amount (mg/system) |
|---|---|
| **Core:** | |
| Ocaperidone | 0.176 |
| Hydroxypropylmethylcellulose | 9.6 |
| Hydroxyethylcellulose 250L | 4.00 |
| Hydroxyethylcellulose 250H | 8.00 |
| Mannitol fine | 61.80 |
| Dextrates hydrated | 61.80 |
| Sodium lauryl sulphate | 1.04 |
| Magnesium stearate | 1.6 |
| Total weight of core | 148.00 |
| Diameter of core | 7.00 mm |
| **Coating** (rate controlling): | |
| Cellulose acetate 320 | 17.0 % |
| Cellulose acetate 398 | 73.0% |
| Hydroxypropylmethylcellulose E15 | 5.0% |
| Polyethylene glycol 8000 | 5.0% |
| Total coating weight/system | 10.4 mg |
| **Ocaperidone coat of 0.044 mg:** | |
| Ocaperidone coat of 0.044 mg | 0.5% |
| Hydroxypropylmethylcellulose E3 | 3.0% |
| Polyvinylpyrrolidone/vinylacetate 64 | 27.0% |
| Additional coating weight/system | 2.50 mg |
| **Total weight of system** | Approx. 161 mg |

### Release rate testing

The release rate was tested on 6 systems as described in the example 1 using USP 2 method. The volume of dissolution used was 100ml. The mean results are shown below:

| Time in hours | % age drug released |
|---|---|
| 2 | 27.4 |
| 4 | 50.7 |
| 6 | 66.1 |
| 8 | 78.2 |
| 10 | 85.2 |
| 12 | 92.9 |
| 18 | 97.9 |
| 24 | 100.2 |

### Stability testing.

The systems were found to be stable with respect to dissolution rate properties after 3 months storage up to 40°C.

## Claims

1. A composition comprising ocaperidone, as an active ingredient, and an effective amount of water-soluble swellable polymers.

2. The composition according to claim 1, comprising a pharmaceutically acceptable carrier.

3. The composition according to claim 1 or 2, wherein the amount of ocaperidone is from 0.05 to 5 % by weight of the total weight of the composition.

4. The composition according to one of claims 1 to 3, wherein ocaperidone is in a finely particulate form.

5. The composition according to one of claims 1 to 4, wherein said polymers are hydrogels of the swellable cellulose ether type, e.g. methyl cellulose, hydroxyethyl cellulose or hydroxypropyl methylcellulose, hydroxypropyl cellulose preferably having a molecular weight higher than 10,000, or mixtures of said swellable hydrophilic polymers.

6. The composition according to one of claims 1 to 4, wherein said polymers are water-soluble polyvinylamides and polyvinylpyrrolidone.

7. The composition according to one of claims 1 to 4, wherein said polymers are selected from the group consisting of hydroxyethyl cellulose, hydroxypropyl cellulose, hydroxypropyl methylcellulose, polyvinylpyrrolidone, and a combination thereof.

8. The composition according to one of claims 1 to 7, wherein the amount of the polymers is from 5 to 50% by weight, most preferably about 15%, based on the total weight of the composition.

9. The composition according to one of claims 1 to 8, wherein it presents a weight ratio ocaperidone:swellable hydrophilic polymers ranging from 1:10 to 1:1000, preferably from 1:50 to 1:500.

10. The composition according to any one of the preceding claims, for treating psychoses, in particular schizophrenia, mania, obsessive compulsive disorders, tourette syndrome, anxiety and bipolar depression.

11. A solid pharmaceutical dosage comprising :
(a) a wall made of a material which is permeable to water and impermeable to the compartments of the ocaperidone-containing core,
(b) a core containing the composition as defined in any one of the preceding claims and a water-soluble compound for inducing osmosis and, optionally, further pharmaceutically acceptable excipients, and
(c) a passageway through the wall (a) for delivering the core components to the environmental body fluid.

12. The solid pharmaceutical dosage according to claim 11, wherein the water-soluble compound for inducing osmosis is selected from the group consisting of sodium chloride, mannitol and dextroses.

13. The solid pharmaceutical dosage according to one of claims 11 and 12, wherein the core of the therapeutic system contains 20-35 % by weight of water-soluble compounds for inducing osmosis based on the total weight of the therapeutic system.

14. The solid pharmaceutical dosage according to any one claims 11-13, wherein the semi-permeable wall is partially or totally coated by a composition comprising ocaperidone.

15. The solid pharmaceutical dosage according to claim 14, wherein said composition comprising ocaperidone is as defined in any one of claims 1-9.

16. The solid pharmaceutical dosage according to any one claims 11-15, for delivering ocaperidone to the rectal tract or to the gastroinstestinal tract.

17. The solid pharmaceutical dosage according to any one claims 11-16, for treating psychosis, in particular schizophrenia, mania, obsessive compulsive disorders, tourette syndrome, anxiety and bipolar depression.
